# EUROPÄISCHE PATENTANMELDUNG

(11) **EP 0 840 123 A1**
(43) Veröffentlichungstag der Anmeldung: **06.05.1998**
(21) Anmeldenummer: 97119064.0
(22) Anmeldetag: 31.10.1997
(51) Int. Cl.: G01N 33/52, G01N 33/543, G01N 33/68

(54) **Vorrichtung zum quantifizierenden und differenzierden Nachweis verschiedener Biomoleküle in einem einzigen Testansatz**

(30) Priorität: 05.11.1996 DE 19645569; 30.05.1997 DE 19722815
(71) Anmelder: Cell Diagnostica, Gesellschaft für angewandte Zellbiologie mbH, 48159 Münster (DE)
(72) Erfinder: Gismann, Michael, 48151 Münster (DE); Hüwel, Stephan, 48151 Münster (DE); Kasten, Elena Dr., 48153 Münster (DE); Michels, Elmar Dr., 48149 Münster (DE); Müller, Olaf, 48161 Münster (DE)
(74) Vertreter: Müller-Boré & Partner Patentanwälte

(57) **Zusammenfassung**

Die Erfindung betrifft eine Vorrichtung zur quantitativen und/oder qualitativen Bestimmung von Analyten in einer zu untersuchenden Lösung. Weiterhin betrifft die Erfindung die Verwendung der Vorrichtung in einem Verfahren zum gleichzeitigen Nachweis von einerseits verschiedenen Antikörpern in Körperflüssigkeiten oder anderen komplexen Analysegemischen, bei welchem verschiedenen Antigene jeweils örtlich voneinander getrennt an ein festes Trägermaterial gebunden werden, mit Körperflüssigkeiten oder anderen komplexen Analysegemischen inkubiert werden und die spezifischen, an die Antigene gebundenen Antikörper mit markierten Detektorantikörpern reagieren und in einer nachfolgenden Reaktion durch eine, aufgrund der Markierung entstehenden Färbung sichtbar gemacht werden. Andererseits können durch dieses Verfahren aber auch verschiedene Antigene nachgewiesen werden, indem spezifische Antikörper an ein festes Trägermaterial gebunden werden. In Abhängigkeit von der Konzentration der nachzuweisenden Antikörper oder Antigene ergibt die Markierung des Nachweisantikörpers eine unterschiedlich starke Farbreaktion auf dem Trägermaterial. Die Erfindung umfaßt insbesondere eine computergestützte Bildauswertung, mit der es möglich ist, das Färbemuster auf dem Träger so auszuwerten, daß die nachzuweisenden Antikörper oder Antigene quantifiziert und differenziert werden können. Die Quantifizierung wird durch auf den Träger aufgebrachte Standards ermöglicht, anhand deren Farbintensität eine Eichgerade erstellt wird, an der dann die Konzentration der Antikörper oder Antigene errechnet wird. Aufgrund des Färbemusters wird zwischen verschiedenen Antikörpern oder Antigenen differenziert.

## Beschreibung

Die Erfindung betrifft eine Vorrichtung zur Verwendung in einem Verfahren zum gleichzeitigen Nachweis verschiedener Antigene und/oder Antikörper in Lösung, insbesondere Körperflüssigkeiten, oder anderen komplexen Analysegemischen. Durch die erfindungsgemäße Vorrichtung ist es insbesondere möglich, die nachzuweisenden Antikörper und/oder Antigene zu quantifizieren und zu differenzieren.

Das Aufbringen von Biomolekülen auf feste Träger ist ein im Stand der Technik ausführlich beschriebenes und vielseitig anwendbares Verfahren. Renart [1] und Towbin [2] beschrieben erstmals 1979 den Transfer elektrophoretisch aufgetrennter Proteine auf eine feste Matrix als *"*Protein blotting*"*. Hierzu werden bevorzugt Nitrocellulose (NC), Celluloseacetat oder synthetische Polymere wie Nylonmembranen als feste Träger verwendet. Das Dot-blotting-Verfahren stellt eine weitere Methode zur Immobilisierung von Biomolekülen auf feste Träger dar [3,4]. Hierbei werden die Analyte durch direktes Auftragen auf der Membran immobilisiert. Das Auftragen kann entweder automatisch, z.B. durch einen Drop-, Line- oder Pattern-Printer, oder auch manuell mit einer Pipette erfolgen. Auch in diesen Fällen werden bevorzugt die oben bereits erwähnten, festen Träger als Immobilisierungsmatrices verwendet. Eine spezielle Anwendung dieser Techniken ist der Dot-ELISA (Enzyme-linked Immunosorbent Assay), der z.B. bei der Detektion geringer Konzentrationen bestimmter Antikörper im Plasma [4] oder dem selektiven Nachweis spezifischer mikrobieller Antigene [5,6] eingesetzt wird. Ein ELISA ist im allgemeinen eine Technik, bei der z.B. ein Antikörper A gegen ein bestimmtes Protein auf einer Mikrotiterplatte immobilisiert wird. Dieser bindet das Antigen, welches dann mit einem zweiten Antikörper B, der ebenfalls das Antigen erkennt und eine Markierung trägt, nachgewiesen werden kann. Im speziellen Fall des Dot-ELISAs wird der Antikörper A oder ein Antigen als Punkt auf einer Trägermembran immobilisiert und durch eine nachgeschaltete immunologische Reaktion nachgewiesen.

Durch den Einsatz solcher Verfahren werden größere Mengen an teuren Antikörperlösungen und Nachweisreagenzien sowie Analytlösung (wie z.B. Patientenserum oder -plasma) benötigt, um die gesamte Filtermembran zu überschichten. Stehen nur geringe Mengen an Analytlösung zur Verfügung, müssen die Dot-ELISA-Membranen in kleine Stücke geschnitten werden, die in je eine Vertiefung einer Mikrotiterplatte passen [3]. Dies Verfahren ist arbeitstechnisch aufwendig und für größere Testreihen nur bedingt geeignet. Ein großer Nachteil dieser Verfahren besteht darin, daß die Ergebnisse nur qualitativ ausgewertet werden können (abgesehen vom ELISA), so daß die Interpretation der Auswertung zu Fehleinschätzungen führen kann. Eine quantitative Auswertung von Dot-ELISA-Membranen ist bisher nur in Form eines Radioimmunoassays beschrieben worden [7]. Dies bedeutet ein erhöhtes Sicherheitsrisiko und damit eine eingeschränkte Verwendbarkeit in der klinischen Diagnostik.

Der vorliegenden Erfindung liegt somit die Aufgabe zugrunde, die dargelegten Nachteile zu beseitigen und eine Vorrichtung zur Verwendung in einem Verfahren bereitzustellen, worin verschiedene Biomoleküle auf einer Trägermatrix immobilisiert werden, dann die daran bindenden Antigene oder Antikörper in einem einzigen Analyseprozeß nachgewiesen werden, und welche somit eine Differenzierung und Quantifizierung erlaubt.

Diese Aufgabe wird durch die in den Ansprüchen angegebenen Merkmale der erfindungsgemäßen Vorrichtung sowie deren Verwendung in einem Verfahren zum quantifizierenden und differenzierenden Nachweis verschiedender Biomoleküle an fester Phase in einem einzigen Testansatz gelöst.

Eine erste bevorzugte Ausführungsform der Erfindung betrifft eine Vorrichtung zur quantitativen und/oder qualitativen Bestimmung von Analyten in einer zu untersuchenden Lösung, umfassend ein erstes Element mit einer Aussparung und einer Vertiefung, wobei die Vertiefung einen Träger aufnehmen kann, an welchem unterschiedliche Biomoleküle jeweils örtlich getrennt voneinander anbringbar sind, und ein zweites Element mit einer Konfiguration, die der Aussparung des ersten Elements angepaßt ist, und durch eine Aussparung eine Vertiefung ausbildet, die am Boden einen Durchbruch aufweist, wobei das zweite Element in die Aussparung des ersten Elements derart eingefügt werden kann, daß es den in der Vertiefung des ersten Elements angeordenten Träger fixiert und einen definierten Reaktionsraum für ein Nachweisverfahren ausbildet.

Diese zweistückige Vorrichtung zur Fixierung des Trägers zeichnet sich besonders dadurch aus, daß in einem von zwei Gehäuseelementen eine Vertiefung angeordnet ist, in welche der feste Träger eingelegt werden kann. Mit dem zweiten Gehäuseelement dieser erfindungsgemäßen Vorrichtung wird der Träger so mit dem ersten Gehäuseelement verbunden, daß er fest, beispielsweise durch Einklemmen oder Kleben, zwischen diesen beiden Elementen angeordnet bzw. fixiert wird. Das zweite Element der Vorrichtung ist mit einer, der Konfiguration der Aussparung des ersten Elements angepaßten Aussparung und einer Durchbrechung ausgestattet. Diese Durchbrechung ist so ausgebildet, daß sie den reaktiven Teil des in der Vertiefung der Aussparung des erstens Elements angeordneten Trägers freiläßt. Die durch Einfügen des zweiten Elements in die Aussparung des ersten Elements ausgebildete Vertiefung stellt einen Reaktionsraum über dem Träger dar, welcher die Analytlösung und die Nachweislösungen vollständig aufnimmt.

Eine weitere bevorzugte Ausführungform der Erfindung betrifft eine einstückige Vorrichtung zur quantitativen und/oder qualitativen Bestimmung von Analyten in einer zu untersuchenden Lösung, umfassend ein erstes Element bzw. einen ersten Abschnitt mit einer Aussparung und einer Vertiefung, wobei die Vertiefung den vorstehend definierten Träger aufnehmen kann, und ein zweites Element bzw. einen zweiten Abschnitt mit einer Konfiguation und/oder Abmessungen, die der Konfiguration und/oder den Abmessungen der Aussparung und/oder der Vertiefung und/oder des Teststreifens angepaßt ist/sind, und durch eine Aussparung eine Vertiefung ausbildet, die am Boden einen Durchbruch aufweist, wobei die ersten und zweiten Elemente bzw. Abschnitte derart angeordnet sind, daß der in der Vertiefung des ersten Elementes bzw. Abschnittes angeordnete Träger fixierbar ist bzw. fixiert ist und daß ein definierter Reaktionsraum für ein Nachweisverfahren ausgebildet ist.

Bei sowohl der einteiligen als auch der zweiteiligen Ausführungsform ist der Durchbruch in dem zweiten Abschnitt oder Element bevorzugt in zumindest einer Richtung kleiner als der Teststreifen, so daß der Teststreifen durch eine Art Vorsprung in der Vertiefung gehalten wird. Bei der zweiteiligen Ausführungsform kann somit z.B. erst der Teststreifen angeordnet werden, und nachfolgend durch das Einfügen des zweiten Elementes festgelegt werden. Bei der einstückigen Ausgestaltung kann der Streifen z.B. über einen gebildeten Vorsprung in die Vertiefung gepreßt werden, so daß der Teststreifen entsprechend einrastet.

Alternativ ist es bevorzugt, daß der Durchbruch im wesentlichen den Abmessungen des Teststreifens und/oder der Aussparung entspricht, so daß der Teststreifen zur Anordnung in der Vorrichtung einfach von oben eingesetzt wird, wobei insbesondere bevorzugt ein Klebstoff zwischengelagert wird, um den Teststreifen bezüglich der Vorrichtung zu fixieren.

Die Aussparung in dem oberen Element oder Abschnitt verjüngt sich bevorzugt zumindest teilweise konisch oder trichterartig nach unten hin zu der Vertiefung, welche den Teststreifen aufnehmen soll, so daß zum einen beim Einsetzen eine automatische Justier- und/oder Positioniereinrichtung bereitgestellt ist und daß zum anderen sich der Reaktionsraum nach oben aufweitet, um das Befüllen mit Reagens zu vereinfachen.

Die Erfindung betrifft ebenfalls die Verwendung der erfindungsgemäßen Vorrichtung in einem Verfahren zur qualitativen und/oder quantitativen Bestimmung von unterschiedlichen Analyten in einer zu untersuchenden Lösung, umfassend die Schritte
(a) des Aufbringens und Immobilisierens von mindestens zwei unterschiedlichen Biomolekülen auf einen Träger, wobei die unterschiedlichen Biomoleküle jeweils örtlich getrennt voneinander aufgebracht werden,
(b) des Inkubierens der auf dem Träger aufgebrachten Biomoleküle mit der zu untersuchenden Lösung, und
(c) des Durchführens einer Nachweisreaktion für Biomolekül/Analyt-Komplexe.

Im ersten Schritt (a) werden spezielle Antigene und/oder Antikörper ("Biomoleküle") auf eine feste Matrix als Träger immobilisiert. Im zweiten Schritt (b) werden Antikörper oder Antigene ("Analyte") aus Körperflüssigkeiten oder anderen komplexen Analysegemischen ("Analytlösung") an die immobilisierten Biomoleküle gebunden und durch eine anschließende Nachweisreaktion gemäß Schritt (c) in Form einer Farbreaktion sichtbar gemacht. Diese Nachweisreaktion beruht auf einer visuellen und/oder computergestützten Bildauswertung, bei welcher die entstandene Färbung auf der Trägermatrix mit Hilfe eines Scanners digitalisiert wird. Ein entsprechendes Computerprogramm wertet dann das digitalisierte Färbemuster so aus, daß die im Schritt (b) gebundenen Analyte quantifiziert und, wenn verschiedene Analyte nachgewiesen werden sollen, auch differenziert werden können.

Bevorzugt werden die verwendeten Biomoleküle so auf einem festen Träger aufgebracht, daß sie irreversibel an den Träger bzw. Trägermatrix gebunden werden. Dies wird dadurch erreicht, daß als Immobilisierungsmatrix ein Trägermaterial verwendet wird, an welches die Biomoleküle zum einen kovalent binden, zum anderen aber auch durch verschiedenartige nicht-kovalente Bindungen (z.B. ionische Wechselwirkungen, Van-der-Waals-Kräfte, hydrophobe Wechselwirkungen) adsorptiv gebunden werden. Das Aufbringen der Biomoleküle geschieht durch ein im Stand der Technik bekanntes Verfahren, bevorzugt mit Hilfe dazu geeigneter Geräte. Zu diesem Zweck eignen sich besonders ein Micro-Drop-System oder ein Line-Printing-System. Es können aber auch andere entsprechende Geräte verwendet werden. Ebenso ist ein manuelles Aufbringen der Biomoleküle mit Hilfe üblicher Pipettierhilfen möglich.

Die Bezeichnung "Biomoleküle" umfaßt zum einen spezifische Antikörper, die distinkte Antigene erkennen, welche dann durch eine Nachweisreaktion detektiert werden, zum anderen aber auch beliebige Antigene, die durch spezifische Antikörper erkannt und die dann ihrerseits nachgewiesen werden.

Die zu detektierenden Antigene oder Antikörper ("Analyte") werden typischerweise aus Körperflüssigkeiten oder anderen komplexen Analysegemischen durch immunologische Verfahren nachgewiesen. Unter Körperflüssigkeiten werden erfindungsgemäß sämtliche Flüssigkeiten verstanden, die aus einem tierischen Körper, insbesondere einem Säuger, vorzugsweise einem Menschen, erhalten werden können. Solche Flüssigkeiten umfassen vorzugsweise Serum, Plasma, Vollblut, Lymphe, Speichel, Ascites und Urin. Weiterhin sind auch solche Flüssigkeiten umfaßt, die aus festen Geweben gewonnen werden können. Unter den "anderen komplexen Analysegemischen" werden insbesondere Fermentationsüberstände, Zellkulturüberstände, Gewebeextrakte sowie Proben aus dem Bereich der Umweltanalytik (Boden und Wasserproben), der Lebensmittelanalytik und der Rückstandsanalytik verstanden. Alle dargelegten Analysegemische können sowohl unverdünnt als auch in verdünnter Form eingesetzt werden. Zur Verdünnung können zu diesem Zweck übliche, im Stand der Technik bekannte Lösungen verwendet werden.

Die zu detektierenden Analyte werden erfindungsgemäß derart nachgewiesen, daß die Trägermatrix mit den aufgebrachten Biomolekülen mit Körperflüssigkeiten oder anderen komplexen Analysegemischen (im folgenden zusammengefaßt und als "Analytlösung" bezeichnet) inkubiert wird, so daß die darin enthaltenen Analyte an die trägerfixierten Biomoleküle binden. Die gebundenen Analyte werden entweder mit spezifischen, markierten Detektorantikörpern, die gegen die Analyte gerichtet sind, oder mit unmarkierten Detektorantikörpern und diese dann mit markierten, gegen die Detektorantikörper gerichteten Nachweisantikörper nachgewiesen, wobei die Markierung in beiden Fällen nicht-radioaktiv ist.

Die Markierung der Antikörper ergibt am Ende der Nachweisreaktion eine Farbreaktion, die in ihrer Intensität direkt proportional zur Konzentration der gebundenen, nachzuweisenden Analyte ist.

Die Markierung der Antikörper erfolgt typischerweise so, daß sie entweder direkt sichtbar werden, wie z.B. bei Kopplung mit kolloidalem Gold, oder die Markierung ist ein Enzym, welches nach Zugabe eines Substrats eine Farbreaktion katalysiert, die dann sichtbar wird. Zu diesem Zweck können Enzyme wie z.B. alkalische Phosphatase, Meerrettich-Peroxidase, Glucose-Oxidase oder sonstige bekannte Markerenzyme verwendet werden. Die Nachweisreaktion kann auch das bekannte Biotin-Streptavidin- bzw. Biotin-Avidin-Verstärkungssystem umfassen.

Aufgrund der Markierung und in Abhängigkeit der Analyte in der Analytlösung entsteht am Ende der Nachweisreaktion ein Färbemuster auf dem Träger, welches entweder visuell oder mit Hilfe einer computergestützten Bildauswertung ausgewertet wird. In beiden Fällen ist sowohl eine qualitative als auch eine quantitative Auswertung möglich, weil die Farbintensität proportional zur Konzentration der gebundenen Analyte ist. Je höher die Analytkonzentration ist, desto intensiver ist auch die Färbung.

Bevorzugt wird eine quantitative Auswertung dadurch ermöglicht, daß auf einem Träger sowohl ein spezifischer Antikörper gegen ein nachzuweisendes Antigen als auch das Antigen selbst als Standard in unterschiedlichen Konzentrationen aufgebracht werden. Selbstverständlich kann auch umgekehrt ein spezifischer Antikörper als Standard und das Antigen für diesen spezifischen Antikörper auf einen Träger aufgebracht werden. Nach der Inkubation mit der Analytlösung und der folgenden Nachweisreaktion werden die Standards entsprechend ihrer Konzentration unterschiedlich stark angefärbt. Ebenso wird der Analyt entsprechend seiner Konzentration angefärbt, so daß ein direkter Vergleich mit dem Standard möglich ist.

In solchen Fällen, in denen nur eine qualitative Aussage getroffen werden soll, ist das Aufbringen von Standards auf den Träger nicht erforderlich.

Ein Färbemuster entsteht dann, wenn erfindungsgemäß verschiedene Biomoleküle an distinkten (d.h. örtlich voneinander getrennten) Stellen auf den Träger aufgebracht werden. Das Muster kann in verschiedenen Formen angelegt sein, so z.B. in Linien oder Punkten. Entscheidend ist hierbei nur, daß an definierten Stellen des Trägers auch nur eine Sorte bzw. Art von Biomolekülen aufgebracht ist.

Bei der rein visuellen Auswertung des Färbemusters kann durch einen Vergleich mit einer externen Schablone eine Zuordnung von angefärbten Bereichen auf dem Träger mit bestimmten Analyten erfolgen. Auf diese Weise ist eine qualitative Aussage möglich. Anhand der Farbintensität des nachgewiesenen Analyten ist darüber hinaus eine Ouantifizierung möglich, wenn Standards mit auf den Träger aufgebracht waren, oder wenn auf einer externen Schablone eine Farbskala angegeben ist, so daß über die Farbintensität eine Konzentrationszuordnung erfolgen kann.

Bei Verwendung einer computergestützten Bildauswertung kann in einem ersten Schritt das Färbemuster mittels eines Scanners digitalisiert werden. Danach wird das digitalisierte Färbemuster mit Hilfe eines speziellen Computerprogramms in 256 Graustufen aufgeschlüsselt, so daß aus jedem angefärbten Bereich des Trägers ein Grauwert errechnet wird. Aufgrund der Standards auf dem Träger wird eine Korrelation zwischen Konzentration und Grauwert (also Farbintensität) ermittelt. Anhand dieser Korrelation kann über den ermittelten Grauwert eines angefärbten Analyten dessen Konzentration berechnet werden. Mit diesem Verfahren ist es darüber hinaus möglich, zwischen verschiedenen Analyten in einem Gemisch zu unterscheiden, wenn an verschiedenen Stellen des Trägers solche Biomoleküle aufgetragen sind, die spezifisch mit nur einem Analyten aus diesem Gemisch reagieren.

Erfindungsgemäß läßt sich somit eine Differenzierung und Quantifizierung von verschiedenen Analyten in einem einzigen Testansatz durchführen.

Weitere Vorteile und Merkmale der Erfindung ergeben sich aus der folgenden beispielhaften Beschreibung einiger derzeit bevorzugter Ausführungsformen unter Bezugnahme auf die Zeichnungen.

Die Figuren zeigen:
Figur 1 ist eine bevorzugte Ausführungsform des Trägers und zeigt ein Allergen-Panel. Auf einer verstärkten Nitrocellulosemembran (1) sind verschiedene Allergene (2) als Biomoleküle sowie Standards (3) in Form von Linien aufgebracht.
Figur 2 zeigt eine bevorzugte Ausführungsform der erfindungsgemäßen Vorrichtung, bestehend aus zwei Gehäuseelementen, von denen das eine als Grundelement (4) und das andere als Einlegerahmen (5) ausgebildet ist.
Figuren 3, 4 und 5 sind Schnittansichten einer bevorzugten Ausführungsform der erfindungsgemäßen Vorrichtung, im wesentlichen der Vorrichtung von Figur 2 entsprechend.
Figuren 6, 7 und 8 zeigen Querschnittsansichten von bevorzugten erfindungsgemäßen Vorrichtungen des einstückigen Typs.

Die vorliegende Erfindung wird nachfolgend anhand eines Beispiels unter Heranziehung der Figuren näher erläutert. Als beispielhafte Anwendung der vorliegenden Erfindung wird die Herstellung, Anwendung und Auswertung eines *in vitro*-Allergietests beschrieben.

### Beispiel

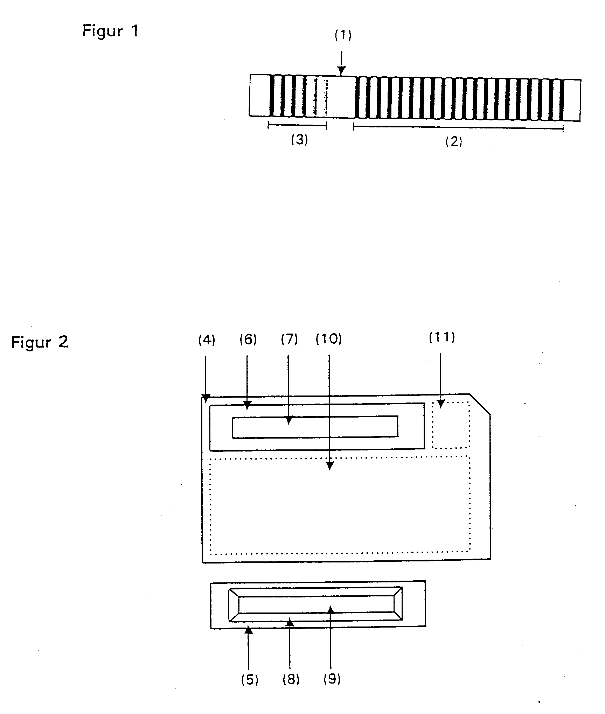

Zunächst wird die Immobilisierung der Biomoleküle an den festen Träger beschrieben werden. Eine Aufsicht ist in Figur 1 dargestellt.

Als Trägermaterial zur Immobilisierung der Biomoleküle wird eine Nitrocellulosemembran (1) mit einer Porenweite von 0,1 µm, die vom Hersteller auf ein festes Trägermaterial aufgebracht worden ist, verwendet. Die Biomoleküle in diesem Anwendungsbeispiel sind Allergene (2) (wie z.B. Milben, Pollen von Gräsern oder Bäumen, Bestandteile aus Nahrungsmitteln oder Hausstaub), die in einer Lösung aus PBS und Glycerin suspendiert vorliegen. Als Standard (3) wird IgE (Immunglobulin E) in verschiedenen Konzentrationen verwendet. Die Konzentrationen der Standards sind so eingestellt, daß sie den international bekannten 6 RAST-Klassen entsprechen. Die so vorliegenden verschiedenen Allergen-Lösungen bzw. Standard-Lösungen werden nun mit Hilfe eines Line-Printers (Bio·Dot Limited, Diddington, England) in Form von Linien auf die Nitrocellulosemembran so aufgetragen, daß ein leiterartiges Muster entsteht (vgl. Figur 1). Nachdem alle Allergene und Standards aufgebracht worden sind, wird die Membran für 1 Stunde in einer 1%igen BSA-Lösung (Rinderserumalbumin) inkubiert, um die nicht mit Allergenen beschichteten Stellen abzusättigen. Die beschichtete Membran wird im folgenden als Allergen-Panel bezeichnet. Die in der Figur 1 dargestellten Banden repräsentieren die auf der Membran aufgetragenen Biomoleküle. Sie sind auf der Membran natürlich transparent und damit nicht sichtbar.

Nachfolgend wird die Herstellung einer bevorzugten Ausführungsform der erfindungsgemäßen Vorrichtung (vgl. Figur 2) und der Zusammenbau von Vorrichtung und Allergen-Panel zum fertigen Allergietest beschrieben.

Die zweiteilige Vorrichtung wird durch ein industrielles Spritzgußverfahren aus einem handelsüblichen Kunststoffmaterial in zwei Teilen hergestellt. Die beiden Teile sind in Form eines größeren Grundelements (4) und eines kleineren Elements als Einlegerahmen (5) ausgebildet. In dem größeren Grundelement (4) ist eine Aussparung (6) eingearbeitet, in die der Einlegerahmen (5) fest eingefügt, beispielsweise geklemmt werden kann. Am Boden dieser Aussparung ist eine Vertiefung (7) eingefügt, die so ausgestaltet ist, daß sie das Allergen-Panel vorzugsweise vollständig aufnimmt. Der Einlegerahmen (5) enthält ebenfalls eine Vertiefung (8), die konisch zuläuft und vorgzusweise der Konfiguration der Aussparung (6) des ersten Elements angepaßt ist, und die eine Durchbrechung (9) aufweist. Der Zusammenbau erfolgt nun so, daß zuerst das Allergen-Panel in die dafür vorgesehene Vertiefung (7) am Boden des größeren Gehäuseelements (4) eingelegt wird, und das Allergen-Panel dann mit dem Einlegerahmen (5), also dem zweiten Gehäuseelement, beispielsweise fest eingeklemmt wird. Der reaktive Teil des Allergen-Panels bleibt durch die Durchbrechung (9) in dem Einlegerahmen (5) frei. Die Vertiefung (8) im Einlegerahmen (5) definiert über dem Allergen-Panel einen Reaktionsraum mit festgelegtem Volumen in den die Analytlösung und die nachfolgend zu verwendenden Lösungen eingefüllt werden können.

Auf dem größeren Gehäuseelement (4) kann ferner eine definierte Fläche (10) vorliegen, die beispielsweise dazu geeignet ist, Etiketten oder Aufkleber aufzunehmen. Insbesondere können solche Etiketten, wie sie aus der klinischen Praxis als Patientenetiketten mit patientenspezifischen Daten und Barcode bekannt sind, verwendet werden. Darüber hinaus kann dieses Gehäuseelement eine weitere Fläche (11) aufweisen, die dazu geeignet ist, produktspezifische Daten, wie Produktbezeichnung, Lot-Nr. oder Haltbarkeitsdatum aufzunehmen.

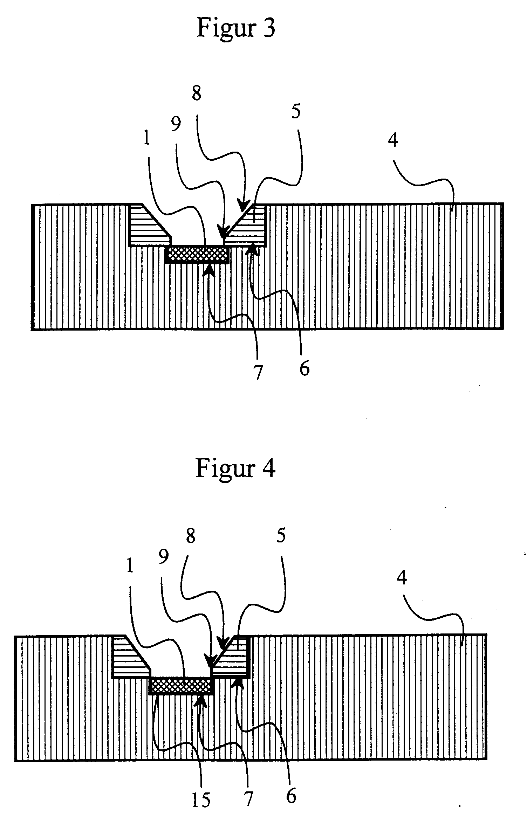

Aus der in Figur 3 gezeigten Schnittansicht läßt sich besonders einfach entnehmen, wie das zweite Element (5) in die Aussparung (6) des ersten Elements eingesetzt ist, und dabei die Trägerplatte (1) in der Vertiefung (7) fixiert. Eine zu Figur 3 senkrecht verlaufende Schnittansicht könnte eine im wesentliche entsprechende Gestalt aufweisen, könnte jedoch auch so vorliegen, wie sie in Figur 4 gezeigt ist, das heißt, daß der von dem Element (5) vorragende Teil, welcher die Trägerplatte (1) fixiert, nicht über den gesamten Perimeter des Durchbruches (9) vorliegen muß, sondern daß vielmehr z.B. lediglich zwei gegenüberliegende Abschnitte derart ausgestaltet sein müssen. Alternativ wäre es auch denkbar, daß lediglich vereinzelte Vorsprünge zur Fixierung der Trägerplatte (1) dienen. Aus den Figuren 3 und 4 ist ersichtlich, daß der Reaktionsraum gebildet ist durch das von dem Durchbruch (9) gebildete Volumen, sowie durch den sich nach oben verbreiternden Raum, welcher durch die Wandungen der Aussparung (8) definiert ist.

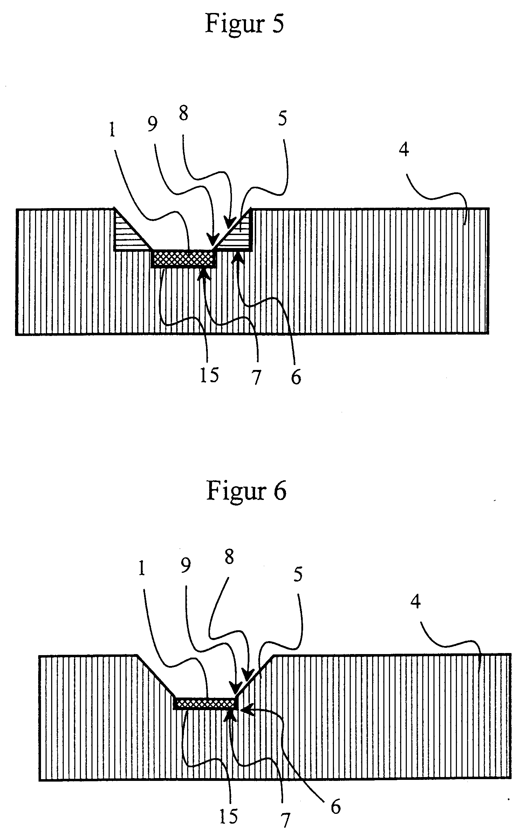

Wie es in Figur 5 gezeigt ist, kann der Reaktionsraum auch ausschließlich durch die Wandungen der Aussparung (8) gebildet sein. Bei den Ausführungsvarianten gemäß den Figuren 4 und 5, bei welchen das zweite Element nicht über die Trägerplatte ragt, kann zur Fixierung der Trägerplatte (1) am Boden der Vertiefung (7) ein Klebstoff vorgesehen sein. Alternativ ist es auch denkbar, daß die Trägerplatte (1) in der Vertiefung (7) mittels Preßpassung oder dergleichen gepaßt ist.

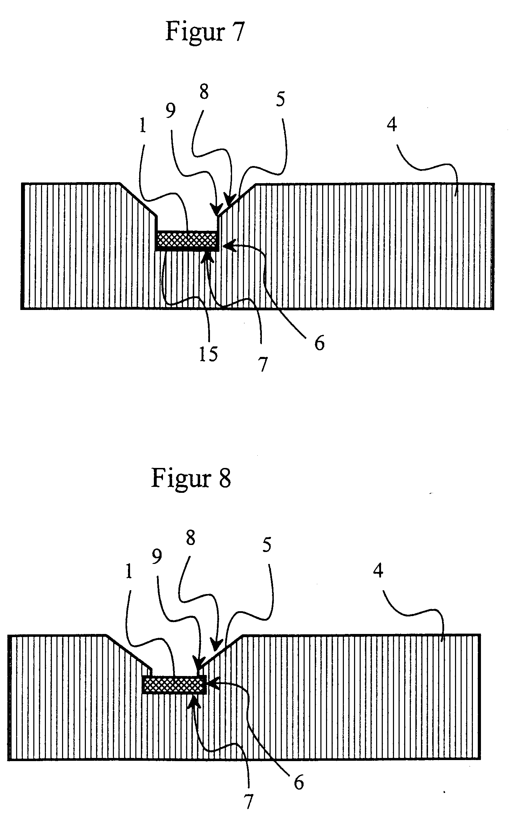

Aus den Schnittansichten der Figuren 3 bis 5 ist es ersichtlich, daß die zwei Elemente (4) und (5) in sehr einfacher Weise ebenfalls als eine einzelne Einheit oder einstückig ausgebildet werden können, wobei insbesondere bevorzugt ein herkömmliches Kunststoff-Spritzgußverfahren zur Herstellung desselben verwendet werden kann. Das Ergebnis einer einstückigen Ausgestaltung der in den Figuren 3 bis 5 gezeigten Ausführungsformen ist in den Figuren 6 bis 8 gezeigt. Hierbei besteht insbesondere folgender Zusammenhang zwischen den einzelnen Figuren, mit der Ausnahme, daß jeweils die Elemente als getrennt oder als einstückig dargestellt sind; Figur 6 enspricht im wesentlichen Figur 5; Figur 7 entspricht im wesentlichen Figur 4; Figur 8 entspricht im wesentlichen Figur 3. Neben der Einstückigkeit ist noch als Unterschied zu erwähnen, daß die Aussparung (6) in den in den Figuren 6 bis 8 gezeigten Ausführungsformen als Seitenwand oder seitliche Aussparung bezüglich der Vertiefung (7) vorgesehen ist.

Die Anordnung des Teststreifens (1) in der Vertiefung (7) erfolgt bei den in den Figuren 6 und 7 gezeigten Ausführungsformen in dem in einem ersten Schritt entweder an der Unterseite des Teststreifens oder auf dem Boden der Vertiefung (7) eine geringe Menge an Klebstoff angebracht wird. Nachfolgend wird der Teststreifen (1) einfach in die Aussparung (8) gelegt, wodurch er automatisch und einfach hin zu der Vertiefung (7) geführt wird. Alternativ zu dem Klebstoff kann auch die Vertiefung (7) entsprechend zu dem Teststreifen ausgebildet sein, so daß eine Passung oder Preßpassung gebildet ist, so daß ein zusätzlicher Klebstoff nicht erforderlich ist.

Bei der in Figur 8 gezeigten Ausführungsform ist die die Vertiefung (7) begrenzende Aussparung (6) seitlich breiter als der Durchbruch (9), so daß der Teststreifen oder die Vorrichtung selbst leicht verformt werden müssen, um den Teststreifen in der Vertiefung (7) anzuordnen.

In jedem Fall ist bei allen gezeigten Ausführungsformen der erfindungsgemäßen Vorrichtung eine Anordnung geschaffen, bei welcher der Teststreifen (1) an einer definierten Position gehalten wird, sei es nun mittels Klebstoff, Eingriff, Wechselwirkung etc.. Über dem Teststreifen ist in jedem Fall ein definierter Reaktionsraum ausgebildet, welcher sich bevorzugt nach oben hin verbreitert, um das Einfügen von Reagenzen zu vereinfachen. Der Reaktionsraum kann begrenzt sein durch Wandungen der Aussparung (8) und/oder des Durchbruches (9).

Derso vollständig zusammengebaute "Allergietest-Chip" wird zur Bestimmung von allergenspezifischem IgE in Serum eingesetzt.

Zu diesem Zweck wird ein definiertes Volumen (in diesem Beispiel 400 µl) unverdünntes oder verdünntes Patientenserum in den Reaktionsraum über der aktiven Fläche der Membran (gebildet durch die Vertiefung (8)) gegeben und für eine bestimmte Zeit, beispielsweise zwischen 45 und 60 Minuten, inkubiert. Sind im Serum allergenspezifische IgE Moleküle vorhanden, so binden diese spezifisch an die auf der Membran aufgebrachten Allergene. Nach der Inkubation wird das Serum von der Membran durch Absaugen oder Abwaschen entfernt und die Membran mit Puffer gewaschen. Zu diesem Zweck wird beispielsweise PBS + 0,5 % Tween verwendet. Es kann aber auch jede andere, zu diesem Zweck geeignete Lösung oder Wasser verwendet werden. Im Anschluß an diesen Waschvorgang wird eine Nachweislösung in den Reaktionsraum pipettiert. Diese Nachweislösung enthält typischerweise Antikörper, die gegen IgE Moleküle gerichtet sind. Der Nachweisantikörper ist entweder mit einem Markerenzym oder mit kolloidalem Gold oder mit Biotin gekoppelt. Als Markerenzyme können typischerweise Meerrettich-Peroxidase, alkalische Phosphatase, Glucose-Oxidase oder andere zu diesem Zweck übliche Enzyme verwendet werden. In dem vorliegenden Ausführungsbeispiel wird bevorzugt ein mit Biotin gekoppelter Nachweisantikörper eingesetzt. Die Nachweislösung wird ebenfalls 45 bis 60 Minuten inkubiert und danach entfernt. Die Membran wird danach wieder gründlich gewaschen und anschließend mit einer Lösung inkubiert, die ein Konjugat aus Streptavidin und alkalischer Phosphatase oder Avidin und alkalischer Phosphatase enthält. Streptavidin bzw. Avidin bindet mit hoher Affinität an das Biotin des Nachweisantikörpers. Nach einer Inkubationszeit von etwa 5 Minuten wird die Membran nochmals gewaschen und danach mit einer Lösung überschichtet, die ein Substrat für das Markerenzym enthält. Als Substrat für die alkalische Phosphatase wird bevorzugt NBT/BCIP (NBT: Nirto-blue Tetrazoliumchlorid, BCIP: 5-Brom-4-chlorindolylphosphat) verwendet, welches durch das Markerenzym in eine farbige Verbindung umgesetzt wird.

Die Intensität der Färbung ist direkt proportional zum gebundenen allergenspezifischen IgE.

Die Standards auf der Membran sind ebenfalls IgE Moleküle, die in 6 unterschiedlichen Konzentrationen aufgetragen sind. Es ist aber auch möglich, weniger als 6 Standardkonzentrationen aufzubringen. Die Standards werden von den Nachweisantikörpern erkannt und im Zuge der weiteren Reaktionen ebenfalls angefärbt. Die Intensität der Färbung ist proportional zur Konzentration des aufgetragenen Standards, so daß eine Eichgerade ermittelt werden kann, an der dann über die Intensität der Färbung der gebundenen allergenspezifischen IgE Moleküle deren Konzentration im Serum bestimmt wird.

Die Färbung auf dem Allergietest-Chip kann sowohl visuell als auch mit elektronischen/optischen Hilfsmitteln ausgewertet werden. Im ersten Fall ist eine qualitative bzw. eine semiquantitative Auswertung möglich. Anhand der Farbintensität kann mit Hilfe der Standards die Konzentration von allergenspezifischen IgE Molekülen abgeschätzt werden.

Im vorliegenden Ausführungsbeispiel wird der gefärbte Allergietest-Chip mit Hilfe eines computergestützten Verfahrens ausgewertet. Hierzu wird das Färbemuster auf dem Allergietest-Chip mit einem Scanner digitalisiert und mit einem Computerprogramm in 256 Graustufen aufgeschlüsselt. Dieses Computerprogramm ordnet dann jedem Balken auf dem Allergen-Panel einen bestimmten Grauwert zu. Anhand der Standards wird eine Eichgerade errechnet (Grauwert gegen IgE Konzentration), mit deren Hilfe dann die Konzentration der allergenspezifischen IgE Moleküle jedes einzelnen Balkens berechnet und einer bestimmten RAST-Klasse zugeordnet wird. Der Begriff RAST-Klasse bezeichnet international festgelegte Konzentrationsbereiche für allergenspezifische IgE Moleküle in Serum.

Im folgenden werden weitere Anwendungsbeispiele aufgeführt, die ebenfalls mit der vorliegenden Erfindung durchgeführt werden können:
- Zytokinzusammensetzung in Körper- und Gewebeflüssigkeiten bzw. in Gewebeextrakten
- Immunoglobulinsubklassenbestimmung
- akute komplexe Hormonmusterbestimmung
- Überprüfung einer Tumormarkerpalette
- Blutgruppenbestimmung (inkl. Rhesusantigenmuster)
- Autoimmunreaktion
- alle denkbaren immunologisch nachweisbaren Antigene, die in der Differentialdiagnostik nachgefragt werden
- Nachweis und Differenzierung von komplexen Enzymaktivitätsmustern
- Nachweis und Differenzierung von unterschiedlichen Inhibitoren
- Nachweis und Differenzierung von immunologisch detektierbaren Umwelttoxinen

### Literatur

[1] Renart, J., Reiser, J. and Stark, G.R., *Proc. Natl*. *Acad*. *Sci*. *USA* 1979, **76**, 3116-3120.
[2] Towbin, H., Staehelin, T. and Gordon, J., *Proc*. *Natl*. *Acad*. *Sci*. *USA* 1979, **76**, 4350-4354.
[3] Hawkes, R., Niday, E. and Gordon, J., *Anal*, *Biochem*. 1982, **119**, 142-147.
[4] Porat, Y.B., Zan-Bar, I. and Ravid, A., *J*. *Immunol*. *Methods* 1995, **180**, 213-218.
[5] Requejo, H.I., Alkim, M., Almeida, R.G., Casagrande, S.T., Cocozza, A.M., Lotufo, J.P., Waetge, A.R. and Rodrigues, J.C., *Rev*. *Inst. Med*. *Trop*. *Sao Paulo* 1994, **36**, 531-537.
[6] Bosompem, K.M., Assoku, R.K. and Nantulya, V.M., *J*. *Immunol*. *Methods* 1995, **187**, 23-31.
[7] Jahn, R., Schiebler, W. and Greengard, P., *Proc*. *Natl*. *Acad*. *Sci*. *USA* 1984, **81**, 1684-1687.

## Patentansprüche

1. Vorrichtung zur quantitativen und/oder qualitativen Bestimmung von Analyten in einer zu untersuchenden Lösung, umfassend ein erstes Element (4) mit einer Aussparung (6) und einer Vertiefung (7), wobei die Vertiefung (7) einen Träger aufnehmen kann, an welchem unterschiedliche Biomoleküle jeweils örtlich getrennt voneinander anbringbar sind, und ein zweites Element (5) mit einer, im wesentlichen der Konfiguration der Aussparung (6) angepaßten Aussparung (8) und einem Durchbruch (9), wobei das zweite Element (5) bezüglich des ersten Elements (4) derart angeordnet ist bzw. werden kann, daß der in der Vertiefung (7) positionierte Träger festlegbar bzw. festgelegt ist, und daß ein definierter Reaktionsraum für ein Verfahren ausgebildet wird.

2. Vorrichtung nach Anspruch 1, bei welcher der Durchbruch (9) in zumindest einer Richtung kleiner als der Teststreifen (1) ist.

3. Vorrichtung nach Anspruch 1, bei welcher die Abmessungen und/oder die Konfiguration des Durchbruches (9) im wesentlichen der Konfiguration bzw. den Abmessungen des Teststreifens (1) und/oder der Vertiefung (7) entspricht/entsprechen.

4. Vorrichtung nach einem der Ansprüche 1 bis 3, bei welcher der Teststreifen (1) mittels eines Klebstoffes (15) am Boden der Vertiefung (7) festlegbar/festgelegt ist.

5. Vorrichtung nach einem der Ansprüche 1 bis 4, bei welcher die Aussparung (8) sich zumindest teilweise konisch oder trichterartig verjüngt.

6. Vorrichtung nach einem der Ansprüche 1 bis 5, bei welchem die Elemente (4) und (5) einstückig ausgebildet sind, insbesondere als ein einstückiges Spritzgußteil.

7. Vorrichtung nach einem der Ansprüche 1 bis 5, bei welchem die zwei Elemente (4, 5) als voneinander getrennte Einheiten, bevorzugt als getrennte Spritzgußteile, ausgebildet sind, wobei das zweite Element (5) in die Aussparung (6) des ersten Elements (4) eingefügt werden kann.

8. Verwendung der Vorrichtung gemäß einem der Ansprüche 1 bis 7 in einem Verfahren zur qualitativen und/oder quantitativen Bestimmung von unterschiedlichen Analyten in einer zu untersuchenden Lösung, umfassend die Schritte
(a) des Aufbringens und Immobilisierens von mindestens zwei unterschiedlichen Biomolekülen auf einen Träger, wobei die unterschiedlichen Biomoleküle jeweils örtlich getrennt voneinander aufgebracht werden,
(b) des Inkubierens der auf dem Träger aufgebrachten Biomoleküle mit der zu untersuchenden Lösung, und
(c) des Durchführens einer Nachweisreaktion für Biomolekül/Analyt-Komplexe.

9. Verwendung nach Anspruch 8, wobei die Nachweisreaktion in Schritt (c) das Inkubieren der Biomoleküle und/oder der Biomolekül/Analyt-Komplexe auf dem Träger mit mindestens einem spezifischen, markierten Detektorantikörper, der gegen die jeweiligen Biomolekül/Analyt-Komplexe oder Analyten gerichtet ist, umfaßt.

10. Verwendung nach Anspruch 8, wobei die Nachweisreaktion in Schritt (c) das Inkubieren der Biomoleküle und/oder der Biomolekül/Analyt-Komplexe auf dem Träger mit mindestens einem spezifischen, unmarkierten Detektorantikörper, der gegen die jeweiligen Biomolekül/Analyt-Komplexe oder Analyten gerichtet ist, und das Inkubieren der Biomolekül/Analyt/Detektorantikörper-Komplexe mit markierten, gegen die Detektorantikörper gerichteten Nachweisantikörper umfaßt.

11. Verwendung nach einem der Ansprüche 8 bis 10, wobei die Nachweisreaktion in Schritt (c) ein Färbemuster auf dem Träger ergibt, welches visuell und/oder mit Hilfe einer computergestützten Bildauswertung ausgewertet wird.

12. Verwendung nach einem der Ansprüche 8 bis 11, wobei die zu untersuchende Lösung aus Säugern, Fermentations- und Kulturüberständen, Gewebeextrakten, Böden, Wasser, Lebensmittel oder Rückständen stammt.
